# EUROPEAN PATENT SPECIFICATION

(11) **EP 3 130 344 B1**
(45) Date of publication and mention of the grant of the patent: **06.03.2019**
(21) Application number: 15764028.5
(22) Date of filing: 19.03.2015
(51) Int. Cl.: A61K 36/752, A61K 36/75, A61P 31/16

(54) **ANTI-INFLUENZA VIRUS COMPOSITION CONTAINING PONCIRUS TRIFOLIATA EXTRACT AS ACTIVE INGREDIENT**
ZUSAMMENSETZUNG GEGEN DAS INFLUENZAVIRUS MIT EXTRAKT AUS PONCIRUS TRIFOLIATA ALS WIRKSTOFF
COMPOSITION CONTRE LE VIRUS DE LA GRIPPE CONTENANT UN EXTRAIT DE PONCIRUS TRIFOLIATA EN TANT QUE PRINCIPE ACTIF

(30) Priority: 19.03.2014 KR 20140032356
(43) Date of publication of application: 15.02.2017
(73) Proprietor: Konkuk University Industrial Cooperation Corp., Seoul 143-701 (KR); KR Biotech Co., Ltd., Gwangjin-gu, Seoul 143-701 (KR)
(72) Inventor: KIM, Young Bong, Goyang-si Gyeonggi-do 410-350 (KR); KIM, Kang Chang, Seoul 137-030 (KR); LEE, Hee-Jung, Seoul 132-030 (KR); YOON, Jong Kwang, Goyang-si Gyeonggi-do 411-410 (KR); KWON, Yong Dae, Seoul 143-130 (KR); HEO, Yoon Ki, Suwon-si Gyeonggi-do 440-302 (KR); HEO, Jae Hyeok., Seoul 131-815 (KR)
(74) Representative: D Young & Co LLP
(86) International application number: PCT/KR2015/002663
(87) International publication number: WO 2015/142069

(56) References cited:
- CN-A- 1 839 976
- KR-A- 20020 033 942
- KR-A- 20020 084 312
- KR-A- 20080 022 315
- KR-A- 20110 008 483
- KR-B1- 100 388 764
- KR-B1- 101 346 412
- ASHWANI SHARMA ET AL: "Drug discovery against H1N1 virus (influenza A virus) via computational virtual screening approach", MEDICINAL CHEMISTRY RESEARCH, BIRKHÄUSER-VERLAG, BOSTON, vol. 20, no. 9, 15 June 2010 (2010-06-15), pages 1445-1449, XP019971299, ISSN: 1554-8120, DOI: 10.1007/S00044-010-9375-5
- NIZAMUTDINOVA IRINA TSOY ET AL: "Hesperidin, hesperidin methyl chalone and phellopterin fromPoncirus trifoliata(Rutaceae) differentially regulate the expression of adhesion molecules in tumor necrosis factor-[alpha]-stimulated human umbilical vein endothelial c", INTERNATIONAL IMMUNOPHARMACOLOGY, vol. 8, no. 5, 1 June 2008 (2008-06-01), pages 670-678, XP029168320, ISSN: 1567-5769, DOI: 10.1016/J.INTIMP.2008.01.011
- Tao Feng: "Anti-human Immunodeficiency Virus-1 Constituents of the Bark of Poncirus trifoliata", , 15 July 2010 (2010-07-15), XP055395901, Retrieved from the Internet: URL:https://www.jstage.jst.go.jp/article/c pb/58/7/58_7_971/_pdf [retrieved on 2017-08-03]
- JANG-HYUN LEE ET AL: "Screening of Antiviral Medicinal Plants against Avian Influenza Virus H1N1 for Food Safety", HAN-GUG CHUGSAN SIGPUM HAG-HOEJI - KOREAN SOCIETY FOR FOOD SCIENCE OF ANIMAL RESOURCES, vol. 30, no. 2, 30 April 2010 (2010-04-30) , pages 345-350, XP055395965, KR ISSN: 1225-8563, DOI: 10.5851/kosfa.2010.30.2.345
- KIM SEONG-YEONG ET AL: "Bioactivity of Trifoliate Orange (Poncirus trifoliate) Seed Extracts", JOURNAL OF FOOD SCIENCE AND NUTRITION, KOREAN INTELLECTUAL PROPERTY OFFICE, vol. 17, no. 2, 30 June 2012 (2012-06-30), pages 136-140, XP053028614, ISSN: 2287-1098, DOI: 10.3746/PNF.2012.17.2.136
- SEONG-YEONG KIM ET AL: "Antimicrobial Activity of Trifoliate Orange (Poncirus trifoliate) Seed Extracts on Gram-Negative Food-borne Pathogens", JOURNAL OF FOOD SCIENCE AND NUTRITION, vol. 17, no. 3, 30 September 2012 (2012-09-30), pages 228-233, XP055293136, ISSN: 2287-1098, DOI: 10.3746/pnf.2012.17.3.228

## Description

### Technical Field

This application claims priority to and the benefit of Korean Patent Application No. 10-2014-0032356 filed in the Korean Intellectual Property Office on 19 March 2014.

The present invention relates to an anti-influenza virus composition containing a *Poncirus trifoliata* seed extract as an active ingredient.

### Background Art

Influenza viruses cause respiratory diseases showing high incidence and mortality rates in humans and animals. There are influenza A virus, influenza B virus, and influenza C virus in influenza viruses, and subtypes of the influenza A virus are H1N1, H2N2, H3N2, H5N1, H7N9, H2N2, H7N7, H1N2, H9N2, H7N2, H7N3, H5N2, and H10N7. Avian influenza has been believed to be spread between birds and pigs. However, in 1997, Hong Kong, among people in contact with birds infected with these viruses, 18 were infected, and six died, and thus the avian influenza has been known to infect humans. Due to avian influenza, in 2003, one of two infected people in Hong Kong and one veterinarian of 83 infected people in Netherland died. All over the world, more than 300 people were infected and at least about 60% cases showed mortality. The H5N1 virus was responsible for the outbreak of avian influenza in Hong Kong, and the H7N7 virus in Netherland. Among these viruses, especially, the highly pathogenic H5N1 influenza A virus changes very fast and is easily spread to other animals. Infected birds come into contact with other chickens or breading ducks or spread avian influenza through excreta, and thus the prevention against avian influenza is not easy. The particular problem is that the avian influenza may be infectious to humans through the infected birds. The infection of humans with avian influenza viruses is more likely to cause variant viruses, and mutagenic variant viruses may cause infection between humans. That is, there is a likelihood of variant viruses spreading between humans.

In April 2009, it was reported that H1N1 virus, which is a new recombinant of influenza A virus, infected 94 humans in Mexico and USA (CDC 2009). Since April of that year, this virus has been continuously spread between humans, and on 11 June 2009, it was classified as the first great pandemic influenza in the 21st century by the World Health Organization (WHO, 2009).

In 2013, the outbreak of avian influenza A (H7N9) caused human infection, resulting in 22 deaths by February 2014 in China.

Influenza A viruses H1N1 and H3N2 are causes of seasonal influenza, and 250,000-500,000 people die from these viruses every year around the world.

Vaccines play an important role in preventing the spreading of influenza viruses between an animal and an animal, between a human and a human, and between an animal and a human, but considering the characteristics of influenza viruses having a high mutation rate, vaccines are designed to target variant influenza viruses, which are expected to become epidemic in the next season, and thus it is impossible for the vaccines to prevent all influenza viruses.

For influenza therapeutics, three types of anti-influenza therapeutics, such as amantadine and rimantadine as M2 ion channel inhibitors, oseltamivir and zanamivir as neuraminidase inhibitors, and ribavirin as an inosine monophosphate dehydrogenase, were approved, and are currently used. These therapeutics are useful in reducing clinical symptoms, but have been restrictively used due to side effects and the appearance of resistant virus variants. Therefore, antiviral therapeutic agents that are effective against influenza are urgently needed.

Plants have developed resisting capacity to viruses through long-term evolution, and thus the plants are receiving a lot of attention as potential resource because they have an excellent antiviral effect against influenza and a high therapeutic effect against the inflammation caused by viral infection.

### Detailed Description of the Invention

### Technical Problem

The present inventors have endeavored to develop a material capable of exhibiting an anti-influenza virus effect from a plant-derived natural extract. As a result, the present inventors established that a *Poncirus trifoliata* seed extract (specifically, a *Poncirus trifoliata* seed extract) has a very excellent anti-viral effect against various influenza viruses, for example, influenza A virus H1N1, H3N2, or H5N1 and influenza B virus, and then completed the present invention.

Therefore, an aspect of the present invention is to provide an anti-influenza virus composition.

Other purposes and advantages of the present invention will become clarified by the following detailed description of invention, claims, and drawings.

### Technical Solution

In accordance with an aspect of the present invention, there is provided an anti-influenza virus composition containing a *Poncirus trifoliata* extract as an active ingredient.

The present inventors have endeavored to develop a material capable of exhibiting an anti-influenza virus effect from a plant-derived natural extract. As a result, the present inventors established that a *Poncirus trifoliata* extract (specifically, a *Poncirus trifoliata* seed extract) has a very excellent anti-viral effect against various influenza viruses, for example, influenza A virus H1N1, H3N2, or H5N1 and influenza B virus.

The *Poncirus trifoliata* extract, which is an active ingredient of the composition of the present invention, can be obtained from a *Poncirus trifoliata* seed extract using various extraction methods known in the art. *Poncirus trifoliata* used in the present invention is a deciduous shrub growing in the south of Gyeonggi-do Province, and robust thorns with 3-5 cm grow in alternation. Leaves growing in alternation have three leaf emergences and leafstalks with some wings. Small leaves are coriaceous, have an obovatus or oval form, are obtuse or acute, and have a length of 25 mm. Flowers bloom in May, are white, acrogenic or ecblastesis, have one or two hanged flowers, five separated sepals and petals, many stamens, and dense hairs in the ovary. Fruits are round, 3 cm in the diameter, aromatic, non-edible, and ripen in September. Seeds are long oval, and 1-1.3 cm in length. The fruits are medicinally used. Seedlings are used for replacement for mandarin trees, and adult trees are received with enthusiasm as a growing hedge in the southern province (Korean Encylopedia of Plants, Lee-changbok, Gyeongmun publishers, 1993).

The *Poncirus trifoliata* extract of the present invention is a *Poncirus trifoliata* seed extract.

The *Poncirus trifoliata* seed extract used in the composition of the present invention may be obtained by treating *Poncirus trifoliata* seeds with various extraction solvents. Preferably, polar solvents or non-polar solvents may be used. Suitable polar solvents may include (i) water, (ii) an alcohol (preferably, methanol, ethanol, propanol, butanol, n-propanol, iso-propanol., n-butanol, 1-pentanol, 2-butoxyethanol, or ethylene glycol), (iii) acetic acid, (iv) dimethyl-formamide (DMFO), and (v) dimethyl sulfoxide (DMSO). Suitable non-polar solvents include acetone, acetonitrile, ethyl acetate, methyl acetate, fluoroalkanes, pentane, hexane, 2,2,4-trimethylpentane, decane, cyclohexane, cyclopentane, diisobutylene, 1-pentene, 1-chlorobutane , 1-chloropentane, o-xylene, diisopropylether, 2-chloropropane, toluene, 1-chloropropane, chlorobenzene, benzene, diethyl ether, diethyl sulfide, chloroform, dichloromethane, 1,2-dichloroethane, aniline, diethyl amine, ether, carbon tetrachloride, and THF.

According to an embodiment of the present invention, examples of the extraction solvent used in the present invention include (a) water, (b) C1-C4 anhydrous or hydrous lower alcohols (methanol, ethanol, propanol, butanol, etc.), (c) mixed solvents of the lower alcohols with water, (d) acetone, (e) ethyl acetate, (f) chloroform, (g) butyl acetate, (h) 1,3- butylene glycol, (i) hexane, and (j) diethyl ether.

According to another embodiment of the present invention, the *Poncirus trifoliata* seed extract is obtained by treating *Poncirus trifoliata* with water, methanol, ethanol, or a combination thereof.

According to a particular embodiment of the present invention, the *Poncirus trifoliata* seed extract of the present invention is obtained by treating *Poncirus trifoliata* with ethanol.

As used herein, the term "extract" has a meaning that is commonly used as a crude extract in the art as described above, and broadly, fractions obtained by additionally fractionating the extract. In other words, the *Poncirus trifoliata* seed extract includes not only ones obtained by using the foregoing extraction solvents but also ones obtained by additionally applying a purification procedure to the same. For example, the fractions obtained by passing the extract through an ultrafiltration membrane with a cut-off value of a predetermined molecular weight, and the fractions obtained through various purification methods that are further carried out, such as separation by various chromatographies (manufactured for separation depending on size, charge, hydrophobicity, or hydrophilicity) are included in the *Poncirus trifoliata* seed extract of the present invention.

The *Poncirus trifoliata* seed extract used in the present invention may be prepared into a powder state by additional procedures, such as vacuum distillation and freeze-drying or spray drying.

As verified in the following examples, the *Poncirus trifoliata* seed extract shows an increase in the survival rate against the influenza virus infection and an effect of the prevention and treatment of influenza in MDCK cells. The *Poncirus trifoliata* seed extract shows an antiviral effect that is equal to or higher than that of Tamiflu, which is widely used as a current influenza therapeutic, and does not affect cytotoxicity even at a high concentration of, especially, 3 mg/mℓ or higher. Therefore, the possibility of using the *Poncirus trifoliata* seed extract as an antiviral drug for preventing or treating diseases caused by infection with influenza A virus H1N1, H3N2, or H5N1 and influenza B virus was confirmed.

According to an embodiment of the present invention, the composition of the present invention has an EC₅₀ value (50% effective concentration) of 0.07-13.7 *µ*g/mℓ with respect to influenza type A H1N1, H3N2, or H5N1 virus and influenza type B viruses.

According to an embodiment of the present invention, the composition of the present invention may be provided as a pharmaceutical composition for preventing or treating diseases caused by influenza A virus H1N1, H3N2, or H5N1 and influenza B virus.

The pharmaceutical composition of the present invention contains, in addition to the foregoing active ingredient, a pharmaceutically acceptable carrier. The pharmaceutically acceptable carrier contained in the pharmaceutical composition of the present invention is usually used at the time of formulation, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present invention may further contain, in addition to the above ingredients, a lubricant, a wetting agent, a sweetening agent, a flavoring agent, an emulsifier, a suspending agent, a preservative, and the like. Suitable pharmaceutically acceptable carriers and preparations are described in detail in Remington's Pharmaceutical Sciences (19th ed., 1995).

A suitable dose of the pharmaceutical composition of the present invention may vary depending on various factors, such as the method for formulation, manner of administration, the age, body weight, gender, and morbidity of the patient, diet, and the time of administration, excretion rate, and response sensitivity. Meanwhile, the oral dose of the pharmaceutical composition of the present invention is preferably 0.001-100 mg/kg (body weight) per day.

The pharmaceutical composition of the present invention may be administered orally or parenterally, and examples of the parenteral administration may include nasal, intravenous, subcutaneous, intramuscular, intraperitoneal, and transdermal injections. The route of administration of the pharmaceutical composition of the present invention is preferably determined according to the kind of applied disease.

The pharmaceutical composition of the present invention may be formulated into a unit dosage form or may be prepared in a multi-dose container by using a pharmaceutically acceptable carrier and/or excipient according to a method that is easily conducted by a person having an ordinary skill in the art to which the present invention pertains. Here, the dosage form may be a solution in an oily or aqueous medium, a suspension, an emulsion, an extract, a powder, granules, a tablet, or a capsule, and may further contain a dispersant or a stabilizer.

The pharmaceutical composition of the present invention may be used together with another pharmaceutically active ingredient that is known to have an antiviral effect in the conventional art, in addition to the *Poncirus trifoliata* seed extract. For example, Lhe pharmaceutical composition of the present invention can improve the antiviral effect by further containing amantadine, rimantadine, oseltamivir, zanamivir, ribavirin, and the like, or combining these.

According to an embodiment of the present invention, the composition of the present invention may be provided in the form of a food composition for preventing or treating diseases caused by influenza A virus H1N1, H3N2, or H5N1 and influenza B virus.

The composition of the present invention, when prepared as a food composition, contains ingredients that are normally added at the time of food manufacturing, in addition to the *Poncirus trifoliata* seed extract, and contains, for example, proteins, carbohydrates, fats, nutrients, seasoning, and flavoring agents. Examples of the foregoing carbohydrate may include normal sugars (monosaccharides, such as glucose and fructose; disaccharides, such as maltose, sucrose, and oligosaccharides; and polysaccharides, such as dextrin and cyclodextrin; typical sugars such as cyclodextrin) and sugar alcohols, such as xylitol, sorbitol, and erythritol. Examples of the flavoring agent may include natural flavoring agents (thaumatin, and stevia extract (e.g., rebaudioside A, glycyrrhizin, etc.)) and synthetic flavoring agents (saccharin, aspartame, etc.). For example, the food composition of the present invention, when is prepared into a drink, may further contain, in addition to the *Poncirus trifoliata* seed extract of the present invention, citric acid, liquefied fructose, sugar, glucose, acetic acid, malic acid, fruit juice, an *Eucommia ulmoides* extract, a jujube extract, and a licorice extract.

The food composition of the present invention may be used for the prevention or treatment of the diseases caused by influenza A virus H1N1, H3N2, or H5N1 and influenza B virus. The food composition according to the present invention may be formulated as a preparation in the same manner as the pharmaceutical composition, and then may be used as a functional food or may be added to various foods. The food to which the composition of the present invention can be added may include, preferably, beverages, vitamin complexes, and health supplement foods. For example, the food composition of the present invention, when is prepared as a beverage, such as a drink, may further contain, in addition to the *Poncirus trifoliata* seed extract, citric acid, liquefied fructose, sugar, glucose, acetic acid, malic acid, fruit juice, extracts of various plants, and the like.

The present invention provides a health functional food comprising a food composition for preventing or treating diseases caused by influenza A virus H1N1, H3N2, or H5N1 and influenza B virus. The health functional food is prepared by adding the *Poncirus trifoliata* seed extract to food materials, such as beverages and teas, or is prepared as a capsule, a powder, or a suspension. The intake of the health functional food means having a particular effect on health, but the health functional food uses a food as a raw material unlike in general drugs, and thus has no side effects that may be caused at the time of long-term administration. The thus obtained health functional food of the present invention is very useful since it can be usually taken. In such a health food, the addition amount of the *Poncirus trifoliata* seed extract cannot be applied in all cases since it depends on the kind of health food, which is an object food, but the addition amount is normally in the range of 0.01-50 wt%, and preferably 0.1-20 wt%, on the basis of the object food. In addition, for a granule, tablet, and capsule type food, the *Poncirus trifoliata* seed extract may be normally added in the range of 0.1-100 wt%, and preferably 0.5-80 wt%. According to an embodiment of the present invention, the health functional food of the present invention may be the form of a beverage.

The food composition or health functional food according to the present invention may be favorably used as a supplement food prior to, or at the same time with, the administration of an antiviral therapeutic, such as amantadine, rimantadine, oseltamivir, zanamivir, or ribavirin.

According to an embodiment of the present invention, the composition of the present invention may be provided in the form of an animal feed additive composition for preventing or treating diseases caused by influenza A virus H1N1, H3N2, or H5N1 and influenza B virus.

The animal feed additive composition of the present invention has an anti-influenza virus effect against for influenza A virus H1N1, H3N2, or H5N1 and influenza B virus, and thus it is expected that the addition of the animal feed additive composition to a livestock feed has an effect of preventing or alleviating the diseases caused by the viruses. The feed used herein means a material which supplies organic or inorganic nutrients needed for retaining the life of livestock and producing milk, meat, eggs, fur, and the like. Feeds may be classified into several types according to the nutritional value, main ingredients, distribution, moisture content, mixed condition, and processing form, and the feed in the present invention includes a coarse feed, a concentrated feed, a supplement feed, a protein feed, a starch feed, a fat feed, and a fibrous feed, but is not limited thereto.

The *Poncirus trifoliata* seed extract contained in the feed additive may be in the form of a highly concentrated liquid, a powder, or a granule. The feed additive composition of the present invention may further contain, in addition to the *Poncirus trifoliata* seed extract, any one, or at least one of organic acids (such as citric acid, fumaric acid, adipic acid, lactic acid, and malic acid), phosphates (such as sodium phosphate, potassium phosphate, acid pyrophosphate, and polyphosphate), and natural antioxidants (such as polyphenol, catechin, α-tocopherol, a rosemary extract, vitamin C, a green-tea extract, a licorice extract, chitosan, tannic acid, and phytic acid).

The animal feed additive composition of the present invention may contain: adjuvant ingredients (such as amino acid, mineral, vitamin, antibiotic material, antibacterial material, antioxidant/antifungal enzyme, or other viable forms of microbial preparations); cereals (e.g., pulverized or crushed wheat, oat, barley, corn, and rice); vegetable protein feeds (e.g., rapeseeds, beans, and sunflower, as main ingredients); animal protein feeds (e.g., blood powder, meat powder, bone powder, and fish powder); and sugar and dairy products (e.g., dried ingredients made of various milk powders and whey powders); lipids (e.g., main ingredients, such as animal lipids optionally liquefied by heating, and vegetable lipids); and additives, such as nutrition supplements, digestion and absorption enhancers, growth promoters, and disease preventing agents).

The feed additive composition of the present invention may be a preparation in a dried form or liquid phase, and may contain a feed additive excipient. Examples of the feed additive excipient may include zeolite, corn flour, or rice bran, but are not limited thereto.

The feed additive composition of the present invention may further contain at least one enzyme preparation. For example, the feed additive composition may contain a lipolytic enzyme such as lipase; phytase decomposing phytic acid into phosphate and inositol phosphate, amylase hydrolyzing α-1,4-glycoside bond contained in starch and glycogen, phosphatase hydrolyzing organic phosphoric acid ester, carboxymethylcellulase decomposing cellulose, xylanase decomposing xylose, maltase hydrolyzing maltose into two glucoses, and invertase hydrolyzing saccharose to produce a glucose-fructose mixture.

The animal feed additive composition of the present invention may be administered alone to an animal or may be administered in combination with other feed additives in an edible carrier, and the feed additives may contain other materials that are known to exhibit an anti-influenza virus effect or to be useful in the prevention or alleviation of respiratory virus infectious diseases including influenza. In addition, the animal feed additive composition may be directly mixed as a top dressing or with an animal feed, or may be easily administered separately from a feed, as a separate oral formulation, or in combination with other ingredients. Usually, as known the art, a daily intake of the animal feed additive composition may be used alone or divisionally.

Examples of the animals, for which the feed additive composition of the present invention can be used, include: livestock, such as edible cow, dairy cow, calf, pig, young pig, sheep, goat, horse, rabbit, dog, and cat; and poultry, such as chick, egg-laying hen, domestic chicken, cock, duck, goose, turkey, quail, and small bird, but are not limited thereto.

The feed including the feed additive containing the *Poncirus trifoliata* seed extract according to the present invention may be obtained by mixing a feed additive containing the *Poncirus trifoliata* seed extract with the feed at an amount of about 1-100 g per 1 kg of the feed on the basis of the dry weight. After the feed mixture is completely mixed, a slightly viscous assembled or granular material is obtained according to the degree of pulverization of ingredients. The resultant material is supplied as a mash, or formed in a desired separate shape for additional processing or packaging. Here, in order to prevent the separation of the feed mixture during storage, water is added to the feed, and then the feed is preferably subjected to a normal palletization, expansion, or extrusion process. Excessive water may be dried and removed.

According to an embodiment of the present invention, the present invention provides a disinfectant composition against influenza A virus H1N1, H3N2, or H5N1, and influenza type B virus, the composition containing a *Poncirus trifoliata* seed extract as an active ingredient.

The composition for influenza virus disinfection of the present invention may further contain, depending on the preparation, a known additive that can be added for killing influenza viruses.

The composition for influenza virus disinfection of the present invention may be prepared as various forms of preparations, such as a liquid, a tablet, a granule, and a powder.

Examples of the known additive that can be added in the composition for disinfection of the present invention include ethanol or isopropyl alcohol for liquid mixing. Especially, ethanol or isopropyl alcohol *per se* has disinfection power and is harmless to humans, and especially, may be added for the purpose of the uniform dispersion of the natural extract having a medicinal action. This alcohol may be contained in, but is not limited to, 1.0 wt% to 80.0 wt%, and preferably 10.0 wt% to 50.0 wt%.

For liquid mixing in the composition of the present invention, purified water may be preferably used for the purpose of dissolution and dispersion of an anti-influenza virus disinfectant. Here, the content of the purified water may be a residual amount after the contents of the added ingredients are determined.

The composition for influenza virus disinfection of the present invention may be prepared as a disinfectant or a detergent. Therefore, the present invention provides a detergent and a disinfectant, which comprise a composition for disinfection of influenza A virus H1N1, H3N2, or H5N1 and influenza B virus, containing a *Poncirus trifoliata* seed extract as an active ingredient. The composition for influenza virus disinfection of the present invention may be commercialized as an anti-respiratory virus disinfectant that is directly sprayed on animal-related facilities and animals, and may be commercialized as an anti-influenza virus disinfectant and detergent for washing hands and instruments in animal-related facilities or in hotels and restaurants requiring sanitation. This disinfectant or detergent may further contain, in addition to the composition for influenza virus disinfection of the present invention, known ingredients required for the disinfectant or detergent.

### Advantageous Effects

Features and advantages of the present invention are summarized as follows:
(i) The present invention provides an anti-influenza virus composition for influenza A virus H1N1, H3N2, or H5N1 and influenza B virus, containing a *Poncirus trifoliata* seed extract as an active ingredient.
(ii) The *Poncirus trifoliata* seed extract of the composition of the present invention inhibits the replication and infection of influenza viruses, thereby exhibiting an excellent anti-viral effect.
(iii) The *Poncirus trifoliata* seed extract, which is an active ingredient of the composition of the present invention, exhibits effects of treating and preventing the infections with influenza A virus H1N1, H3N2, or H5N1 and influenza B virus, has no cytotoxicity even at high concentrations, unlike Tamiflu, and exhibits its effects at lower concentrations.
(iv) The composition of the present invention can be used as a pharmaceutical composition for treating or preventing the diseases caused by influenza viruses, and can be developed as a functional food composition, an animal feed additive composition, or a disinfectant composition for alleviating or preventing the diseases caused by influenza viruses.

### Brief Description of the Drawings

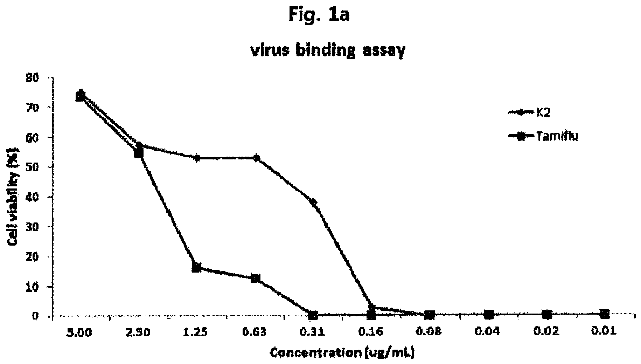

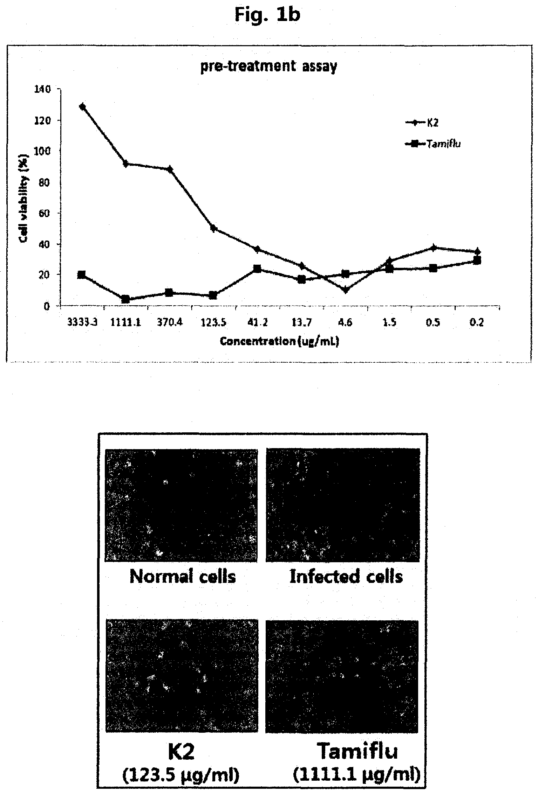

Fig. 1a is a graph showing an inhibitory effect of a *Poncirus trifoliata* seed extract on the binding of influenza A virus H1N1 to MDCK cells. Fig. 1b is a graph showing cell viability of MDCK cells according to the concentration when MDCK cells were treated with *Poncirus trifoliata* seed extract or Tamiflu at indicated concentrations and then infected with influenza A virus H1N1. Attached are images of the cells treated with *Poncirus trifoliata* seed extract or Tamiflu at the indicated concentrations in the test.

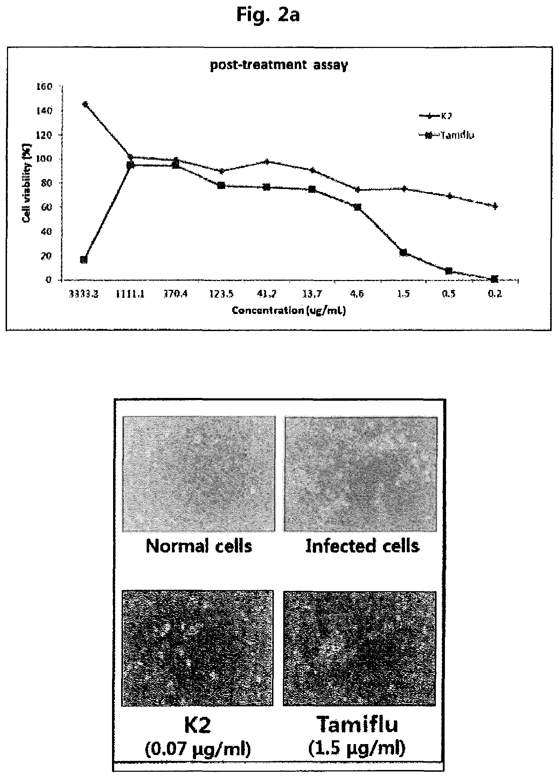

Figs. 2a to 2d are graphs showing cell viability of MDCK cells according to the concentration when MDCK cells infected with influenza A virus H1N1(a), H5N1(b), H3N2(c), or influenza B virus (d) were treated with *Poncirus trifoliata* seed extract or Tamiflu at the indicated concentrations. Attached are images of the cells infected with influenza A virus H1N1 and then treated with *Poncirus trifoliata* seed extract or Tamiflu at the indicated concentrations in the test.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLES

### Materials and Methods

### Preparation of Poncirus trifoliata seed extract

100 g of pulverized dried *Poncirus trifoliata* seeds were extracted in 1 L of ethanol for 2 days, followed by filtration, and then the collected *Poncirus trifoliata* seed ethanol extract was vaporized under reduced pressure for 2-3 days, followed by freeze-drying, to give an extract.

### Cell culture

The MDCK cell line used in the test was incubated in conditions of 37□ and 5% CO₂. The cells were maintained in an Eagle's minimum essential medium (EMEM, Gibco) containing 10% fetal bovine serum (FBS, Hyclone Thermo Scientific) and 1% penicillin-streptomycin solution (Gibco).

### Investigation on cell binding of influenza virus

MDCK cells dispensed in the 96-well plate were cultured at 4□ for 1 hour. The cell line was infected with a mixture of 100 TCID₅₀ of influenza virus H1N1 and different concentrations of *Poncirus trifoliata* seed extract or Tamiflu, cultured at 4□ for 3 hours, washed twice with PBS, and again incubated at 37□. After 48 hours, cell viability was measured. The cell viability was directly observed under a microscope, and the cell viability was again quantified and digitized using MTT assay kit (Dae ill lab servers co. Seoul. Korea). EC₅₀ value is a concentration of an antiviral drug at a section in which cell viability is 50%.

### Investigation on influenza virus infection preventive effect of Poncirus trifoliata seed extract

1 × 10⁴ MDCK cells were dispensed in each well of the 96-well plate, incubated at 37□ for 16 hours, and washed twice with PBS, and then the MDCK cells were treated with different concentrations of *Poncirus trifoliata* seed extract or Tamiflu. The cells were again incubated at 37□ for 6 hours, and washed twice with PBS, and then the cells were infected with 100 TCID₅₀ of influenza virus H1N1, followed by incubation at 37□. After two hours, the cells were washed three times with PBS to remove uninfected virus. Then, the cells were put in a virus growth medium (EMEM, 0.3% BSA, 1% P/S, 0.0005% tyrosine), followed by incubation at 37□ for 48 hours, and then the cytopathic effect (CPE) was observed.

### Investigation on antiviral activity of Poncirus trifoliata seed extract

1 × 10⁴ MDCK cells were dispensed in each well of the 96-well plate, incubated at 37□ for 16 hours, and washed twice with PBS, and then the MDCK cells were infected with 100 TCID₅₀ of influenza A virus H5N1, H1N1, or H3N2 or influenza B virus, respectively, at 37□ for 2 hours, followed by washing twice with PBS. The cells infected with the influenza virus were treated with different concentrations of *Poncirus trifoliata* seed extract or Tamiflu, and then incubated at 37□ for 48 hours. The antiviral effect of the *Poncirus trifoliata* seed extract was expressed by the cytopathic effect (CPE) inhibitory effective concentration 50% (EC₅₀) value. The cytotoxic concentration 50% value (CC₅₀) of the *Poncirus trifoliata* seed extract was determined based on the cellular morphological transformation. The anti-influenza virus capacity of the *Poncirus trifoliata* seed extract was expressed as selectivity index (SI), which is the CC₅₀ value divided by the EC₅₀ value.

### Results

### Virus binding inhibitory effect of Poncirus trifoliata seed extract

A cell line was infected with a mixture of influenza virus H1N1 and different concentrations of *Poncirus trifoliata* seed extract or Tamiflu, followed by incubation. As a result, Tamiflu showed no antiviral effect since all the cells were infected with the virus, while the *Poncirus trifoliata* seed extract showed an EC₅₀ value of 0.25 *µ*g/mℓ, indicating an effect of inhibiting the binding of the virus to cells (Fig. 1a).

### Virus preventive effect of Poncirus trifoliata seed extract

The MDCK cells cultured by the test method above were treated with different concentrations of *Poncirus trifoliata* seed extract or Tamiflu, and then the cells were infected with influenza virus H1N1, followed by incubation at 37□ for 48 hours, and the CPE of cells was observed. As a result, all the cells treated with Tamiflu were dead due to infection with the virus, while the EC₅₀ value was 123.5 *µ*g/mℓ for the treatment with *Poncirus trifoliata* seed extract (Fig. 1b). In addition, as a result of observing the cells using a microscope, the treatment with Tamiflu showed an infected cell state, while the treatment with 123.5 *µ*g/mℓ *Poncirus trifoliata* seed extract showed that the cells were not infected with the virus, similar to normal cells (Fig. 1b).

These results show that the *Poncirus trifoliata* seed extract, unlike Tamiflu, inhibited the cell infection by viruses, suggesting the possibility that the *Poncirus trifoliata* seed extract has a high preventive effect against influenza.

### Antiviral activity of Poncirus trifoliata seed extract

MDCK cells were infected with influenza A virus H1N1, H3N2, or H5N1, or influenza B virus by the test method above, and then treated with different concentrations of *Poncirus trifoliata* seed extract or Tamiflu, to analyze antiviral activity (post-treatment assay). The antiviral effect of the *Poncirus trifoliata* seed extract exhibited the CPE inhibitory effective concentration 50% value (EC₅₀) by viral infection, and the cytotoxic concentration 50% value (CC₅₀) of the *Poncirus trifoliata* seed extract was determined based on the cellular morphological transformation. The EC₅₀ value and CC₅₀ value were calculated by Reed and Muench methods. The anti-influenza virus capacity of the *Poncirus trifoliata* seed extract was expressed as selectivity index (SI), which is the CC₅₀ value divided by the EC₅₀ value. Table 1 shows comparative results of anti-influenza viral activity of Tamiflu and the *Poncirus trifoliata* seed extract on influenza A virus H1N1, H3N2, or H5N1, or influenza B virus in MDCK cells.

**[Table 1]**

| Virus strain | Compound | CC₅₀ (*µ*g/mℓ) | EC₅₀ (*µ*g/mℓ) | SI |
|---|---|---|---|---|
| H1N1 | *Poncirus trifoliata* seed extract | 3333.3 | 0.07 | 4761 |
| H1N1 | Tamiflu | 1111.1 | 3.5 | 317 |
| H3N2 | *Poncirus trifoliata* seed extract | 3333.3 | 1.5 | 2222 |
| H3N2 | Tamiflu | 1111.1 | 3.5 | 317 |
| H5N1 | *Poncirus trifoliata* seed extract | 3333.3 | 0.5 | 6666 |
| H5N1 | Tamiflu | 1111.1 | 1.5 | 740 |
| Influenza B virus | *Poncirus trifoliata* seed extract | 3333.3 | 13.7 | 243 |
| Influenza B virus | Tamiflu | 1111.1 | 13.7 | 81 |

As can be seen from table 1, as for Tamiflu used as a positive control, the CC₅₀ value was 1111.1 *µ*g/mℓ, and the EC₅₀ value was 3.5 *µ*g/mℓ for H1N1 and H3N2, 1.5 *µ*g/mℓ for H5N1, and 13.7 *µ*g/mℓ for the influenza B virus. As for the *Poncirus trifoliata* seed extract, the cytotoxicity was not shown at 3333.3 *µ*g/mℓ, and the EC₅₀ value was 0.07 *µ*g/mℓ for H1N1, 1.5 *µ*g/mℓ for H3N2, 0.5 *µ*g/mℓ for H5N1, and 13.7 *µ*g/mℓ for influenza B virus.

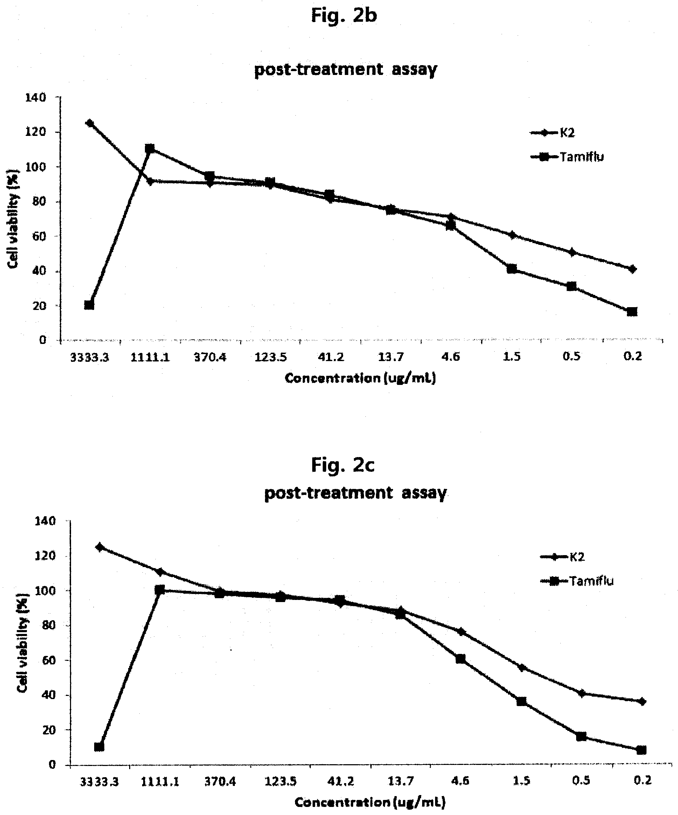

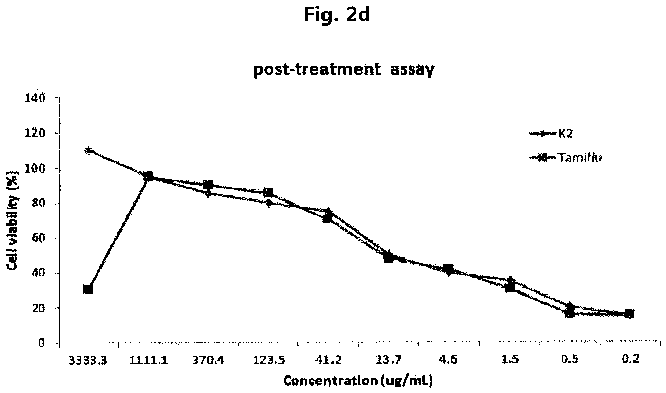

In the results with respect to H1N1 infected strain, as for Tamiflu, the EC₅₀ value showing an anti-viral effect of 50% or more was observed at 3.5 *µ*g/mℓ, and as for the *Poncirus trifoliata* seed extract, the 50% antiviral effect (EC₅₀) was showed at 0.2 *µ*g/mℓ or more. In addition, as a result of observing the cells by a microscope, as for Tamiflu, many cells were infected and dead, while even the treatment with 0.07 *µ*g/mℓ *Poncirus trifoliata* seed extract showed an anti-viral effect (Fig. 2a). In the results with respect to the H5N1 infected strain, as for Tamiflu, the EC₅₀ value was observed at 1.5 *µ*g/mℓ*,* and as for the *Poncirus trifoliata* seed extract, the 50% antiviral effect (EC₅₀) was showed at 0.5 *µ*g/mℓ (Fig. 2b). In the results with respect to the H3N2 infected strain, as for Tamiflu, the EC₅₀ value was observed at 3.5 *µ*g/mℓ, and as for the *Poncirus trifoliata* seed extract, the 50% antiviral effect (EC₅₀) was showed at 1.5 *µ*g/mℓ (Fig. 2c) . In the results with respect to the influenza B virus infected strain, as for both Tamiflu and the *Poncirus trifoliata* seed extract, the 50% antiviral effect was showed at 13.7 *µ*g/mℓ (Fig. 2d).

Overall, the above results show that the *Poncirus trifoliata* seed extract have an antiviral activity, which is as high as that of Tamiflu, on influenza A viruses H1N1, H3N2, and H5N1, and influenza B virus. These results suggest the possibility that the *Poncirus trifoliata* seed extract has a relatively high treatment effect on influenza viruses.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A composition comprising a *Poncirus trifoliata* seed extract for use in the prevention or treatment of influenza virus infections.

2. The composition for use of claim 1, wherein the influenza virus is influenza A virus, influenza B virus, or influenza C virus.

3. The composition for use of claim 1, wherein the influenza A virus is H1N1, H3N2, H5N1, or H7N9.

4. The composition for use of claim 1, wherein the composition is a pharmaceutical composition for preventing or treating diseases caused by influenza viruses.

5. The composition for use of claim 1, wherein the composition is a functional food composition for preventing or treating diseases caused by influenza viruses.

6. The composition for use of claim 1, wherein the composition is an animal feed additive composition for preventing or treating diseases caused by influenza viruses.

7. The composition for use of claim 1, wherein the composition is a disinfectant composition for preventing diseases caused by influenza viruses.

## Patentansprüche

1. Zusammensetzung, umfassend ein *Poncirus-trifoliata*-Samenextrakt zur Verwendung bei der Prävention oder Behandlung von Influenzavirusinfektionen.

2. Zusammensetzung zur Verwendung nach Anspruch 1, wobei es sich bei dem Influenza-Virus um Influenza-A-Virus, Influenza-B-Virus oder Influenza-C-Virus handelt.

3. Zusammensetzung zur Verwendung nach Anspruch 1, wobei es sich bei dem Influenza-A-Virus um H1N1, H3N2, H5N1 oder H7N9 handelt.

4. Zusammensetzung zur Verwendung nach Anspruch 1, wobei es sich bei der Zusammensetzung um eine pharmazeutische Zusammensetzung zur Prävention oder Behandlung von durch Influenzaviren verursachte Krankheiten handelt.

5. Zusammensetzung zur Verwendung nach Anspruch 1, wobei es sich bei der Zusammensetzung um eine Functional-Food-Zusammensetzung zur Prävention oder Behandlung von durch Influenzaviren verursachte Krankheiten handelt.

6. Zusammensetzung zur Verwendung nach Anspruch 1, wobei es sich bei der Zusammensetzung um eine Tierfutterzusatzstoffzusammensetzung zur Prävention oder Behandlung von durch Influenzaviren verursachte Krankheiten handelt.

7. Zusammensetzung zur Verwendung nach Anspruch 1, wobei es sich bei der Zusammensetzung um eine Desinfektionsmittelzusammensetzung zur Prävention von durch Influenzaviren verursachte Krankheiten handelt.

## Revendications

1. Composition comprenant un extrait de graines de *Poncirus trifoliata,* pour une utilisation dans la prévention ou le traitement d'infections par des influenza virus.

2. Composition pour une utilisation selon la revendication 1, **caractérisée en ce que** l'influenza virus est l'influenza virus A, l'influenza virus B, ou l'influenza virus C.

3. Composition pour une utilisation selon la revendication 1, **caractérisée en ce que** l'influenza virus A est H1N1, H3N2, H5N1, ou H7N9.

4. Composition pour une utilisation selon la revendication 1, **caractérisée en ce que** la composition est une composition pharmaceutique destinée à la prévention ou au traitement de maladies provoquées par des influenza virus.

5. Composition pour une utilisation selon la revendication 1, **caractérisée en ce que** la composition est une composition alimentaire fonctionnelle destinée à la prévention ou au traitement de maladies provoquées par des influenza virus.

6. Composition pour une utilisation selon la revendication 1, **caractérisée en ce que** la composition est une composition d'additif pour aliment pour animaux destinée à la prévention ou au traitement de maladies provoquées par des influenza virus.

7. Composition pour une utilisation selon la revendication 1, **caractérisée en ce que** la composition est une composition désinfectante destinée à la prévention de maladies provoquées par des influenza virus.
